# EUROPÄISCHE PATENTSCHRIFT

(11) **EP 3 579 852 B1**
(45) Veröffentlichungstag und Bekanntmachung des Hinweises auf die Patenterteilung: **20.11.2024**
(21) Anmeldenummer: 18708059.3
(22) Anmeldetag: 13.02.2018
(51) Int. Cl.: A61K 35/747, A61K 35/52, A61P 15/08

(54) **VERWENDUNG VON LACTOBACILLUS BUCHNERI ZUR ERHÖHUNG DER FERTILITÄT EINES WEIBLICHEN SÄUGETIERS**
USE OF LACTOBACILLUS BUCHNERI FOR INCREASING THE FERTILITY OF A FEMALE MAMMAL
UTILISATION DE LACTOBACILLUS BUCHNERI POUR AUGMENTER LA FERTILITÉ D'UN MAMMIFÈRE FEMELLE

(30) Priorität: 13.02.2017 DE 102017202233
(43) Veröffentlichungstag der Anmeldung: 18.12.2019
(73) Patentinhaber: Freie Universität Berlin, 14195 Berlin (DE)
(72) Erfinder: GABLER, Christoph, 14513 Teltow (DE); PETER, Sarah, 10249 Berlin (DE); EINSPANIER, Ralf, 14532 Kleinmachnow (DE)
(74) Vertreter: Maikowski & Ninnemann Patentanwälte Partnerschaft mbB
(86) Internationale Anmeldenummer: PCT/EP2018/053542
(87) Internationale Veröffentlichungsnummer: WO 2018/146332

(56) Entgegenhaltungen:
- DATABASE EMBASE [online] ELSEVIER SCIENCE PUBLISHERS, AMSTERDAM, NL; 3 April 2018 (2018-04-03), PETER S ET AL: "Influence of intrauterine administration of Lactobacillus buchneri on reproductive performance and pro-inflammatory endometrial mRNA expression of cows with subclinical endometritis", Database accession no. EMB-621535404
- PETER S ET AL: "Influence of intrauterine administration of Lactobacillus buchneri on reproductive performance and pro-inflammatory endometrial mRNA expression of cows with subclinical endometritis", JOURNAL FUR REPRODUKTIONSMEDIZIN UND ENDOKRINOLOGIE 2018 KRAUSE UND PACHERNEGG GMBH NLD, vol. 15, no. 1, 2018, pages 30 CONF 20180221 to 20180223 Hannover - 51st Annu, ISSN: 1810-9292
- DENG, Q. ET AL.: "Intravaginally administered lactic acid bacteria expedited uterine involution and modulated hormonal profiles of transition dairy cows", JOURNAL OF DAIRY SCIENCE, vol. 98, 2015, pages 6018 - 6028
- BARBONETTI, A. ET AL.: "Effect of vaginal probiotic lactobacilli on in vitro-induced sperm lipid peroxidation and its impact on sperm motility and viability", FERTILITY AND STERILITY, vol. 95, 2011, pages 2485 - 2488
- DATABASE WPI Week 199721, Derwent World Patents Index; AN 1997-234299, XP002781248
- S. LIU ET AL: "Complete Genome Sequence of Lactobacillus buchneri NRRL B-30929, a Novel Strain from a Commercial Ethanol Plant", JOURNAL OF BACTERIOLOGY, vol. 193, no. 15, 1 August 2011 (2011-08-01), US, pages 4019 - 4020, XP055477398, ISSN: 0021-9193, DOI: 10.1128/JB.05180-11
- GAERTNER MARTINA A ET AL: "Detection and Characterisation of Lactobacillus spp. in the Bovine Uterus and Their Influence on Bovine Endometrial Epithelial Cells In Vitro", PLOS ONE, vol. 10, no. 3, March 2015 (2015-03-01), XP002781249
- SHELDON, I. M.; LEWIS, G. S.; LEBLANC, S.; GILBERT, R. O.: "Defining postpartum uterine disease in cattle", THERIOGENOLOGY, vol. 65, 2006, pages 1516 - 1530, XP027930298
- GILBERT, R. O.; SHIN, S. T.; GUARD, C. L.; ERB, H. N.; FRAJBLAT, M.: "Prevalence of endometritis and its effects on reproductive performance of dairy cows", THERIOGENOLOGY, vol. 64, 2005, pages 1879 - 1888, XP027746784
- PETER S ET AL: "Influence of intrauterine administration of Lactobacillus buchneri on reproductive performance and pro-inflammatory endometrial mRNA expression of cows with subclinical endometritis.", SCIENTIFIC REPORTS, vol. 8, no. 1, 3 April 2018 (2018-04-03), pages 1 - 13, XP002781250, ISSN: 2045-2322, DOI: 10.1038/S41598-018-22856-Y

## Beschreibung

Die vorliegende Erfindung betrifft die Verwendung von *Lactobacillus buchneri* zur Erhöhung der Fertilität eines weiblichen Säugetiers gemäß dem Oberbegriff des Anspruchs 1, die Verwendung von *Lactobacillus buchneri* als Bestandteil eines Spermienpräparats gemäß dem Oberbegriff des Anspruchs 8 sowie ein Spermienpräparat mit *Lactobacillus buchneri* gemäß dem Oberbegriff des Anspruchs 9.

Rinder sind ökonomisch gesehen die wichtigsten Nutztiere in der deutschen Landwirtschaft, denn etwa die Hälfte aller Landwirte in Deutschland hält Rinder, um Milch oder Fleisch zu produzieren. Insgesamt verteilen sich deutschlandweit ca. 12,5 Millionen Rinder auf ca. 163.000 Betriebe. Die Milch- und Fleischproduktion sichert insgesamt ein Viertel des landwirtschaftlichen Produktionswertes. Dieser betrug 2013 rund 15 Milliarden Euro, wobei mehr als 11 Milliarden Euro auf Milch und Milchprodukte entfielen. In der Europäischen Union ist Deutschland mit 4,2 Millionen Milchkühen und einer jährlichen Produktion von ca. 30 Millionen Tonnen Milch der größte Milcherzeuger.

Sinkende Fertilitätsraten von Milchkühen mit damit verbundenen großen wirtschaftlichen Verlusten stellen aktuell ein großes Problem in der Landwirtschaft und der Veterinärmedizin dar. Als Ursache dafür wird der Entzündung der Gebärmutterschleimhaut (Endometritis) eine große Rolle zugeschrieben (Sheldon et al., 2014). Die Endometritis tritt meist nach dem Abkalben durch das Aufsteigen pathogener Keime in den Uterus der Kuh durch die geöffnete Zervix auf. Die Prävalenz dieser Krankheit liegt je nach Herde und Zeitpunkt bei 20-90% (LeBlanc et al., 2011). Endometritis wird überwiegend durch die intrauterine Anwendung lokal wirkender Antibiotika und aseptischer Lösungen behandelt, aber oft mit nicht zufriedenstellenden Ergebnissen.

Die Gesamtmenge der von Tierärzten abgegebenen Antibiotika in Deutschland lag im Jahre 2011 bei 1734 Tonnen (Quelle: Bundesamt für Verbraucherschutz). Da jeder Antibiotikaeinsatz Resistenzentwicklungen pathogener Keime fördern kann und oft unerwünschte Wartezeiten auf Fleisch (essbares Gewebe) und Milch zur Folge hat, sollte ein vorrangiges Ziel in der Veterinärmedizin die Beschränkung des Antibiotikaverbrauchs auf ein nötiges Minimum sein. Ferner hat ein Antibiotika-Einsatz auch die unerwünschte Nebenwirkung, dass die kommensale Mikroflora des Uterus zerstört wird.

Aus der RU 2 067 450 C1 ist ein Verfahren zur Behandlung der klinischen Endometritis bei Kühen bekannt, bei dem der Stamm "Kirov-4" des *Lactobacillus buchneri* intrauterin appliziert wird.

Bhandari und Prabha (2015) beschreiben die intravaginale Verwendung von Laktobazillen, insbesondere des Stamms *L. plantarum* 2621, zur Erhöhung der Fertilität bei Mäusen.

Deng et al. (2015) beschreibt die intravaginale Verwendung verschiedener Milchsäurebakterien (LAB) zur peripartalen Behandlung von Rindern. Es wurden die Stämme *Lactobacillus sakei* FUA3089, *Pediococcus acidilactici* FUA3138 und *Pediococcus acidilactici* FUA3140 verwendet. Eine Behandlungsgruppe erhielt diese Milchsäurebakterien lediglich vor der Abkalbung, während eine zweite Behandlungsgruppe sowohl vor als auch nach der Abkalbung mit den Milchsäurebakterien behandelt wurde. Bei der einen Behandlungsgruppe konnte die Güstzeit um 40 Tage (von 150 Tage auf 110 Tage) verkürzt werden. Bei der anderen Behandlungsgruppe (mit denselben Milchsäurebakterien) konnte eine derartige Reduktion der Güstzeit hingegen nicht beobachtet werden.

Otero et al. (2006) beschreibt die Verwendung von 76 Lactobacillus-Stämmen zur Behandlung von Kühen und zur Verhinderung der Ausbildung einer Metritis nach der Abkalbung. Die verwendeten Stämme wurden als *Lactobacillus fermentum, Lactobacillus gasseri* und *Lactobacillus rhamnosus* identifiziert.

Barbonetti et al. (2005) beschreibt eine Kombination dreier Lactobacillus-Stämme (*L. brevis, L. salivarius* und *L. plantarum*) zum Schutz von Spermien vor oxidativem Stress.

Scheldon et al. (2006) beschreibt und definiert verschiedene postpartale uterine Erkrankungen in Kühen, nämlich die puerperale Metritis, die klinische Endometritis, die subklinische Endometritis und die Pyometra.

Gilbert et al. (2005) betrifft die gemeinsame Diagnose von klinischer Endometritis und subklinischer Endometritis mittels zytologischer Methoden. Dabei wurde eine Prävalenz der zytologisch diagnostizierten Endometritis von 53 % ermittelt. Im letzten Satz des letzten Absatzes des Diskussionsteils auf Seite 1887 dieser Veröffentlichung wird ausgeführt, dass eine zytologische Diagnose als Grundlage für Studien zur Prävention und Behandlung einer Endometritis dienen kann.

Liu et al. (2011) beschreibt die Genomsequenz eines spezifischen Stamms von *Lactobacillus buchneri.*

Gärtner et al. (2015) beschreibt, dass *Lactobacillus buchneri* - im Unterschied zu anderen *Lactobacillus-Spezies* - keinen Einfluss auf die mRNA-Expression pro-inflammatorischer Parameter in endometrialen Epithelzellen hat. Demgegenüber wurde in dieser Veröffentlichung für *Lactobacillus ruminis* und *Lactobacillus amylovorus* die Eignung, eine endometriale Immunantwort zu stimulieren, identifiziert.

Der vorliegenden Erfindung liegt die Aufgabe zugrunde, eine antibiotikafreie Behandlung weiblicher Säugetiere bereitzustellen, durch die die Fertilität dieser Säugetiere erhöht wird.

Diese Aufgabe wird mit einer neuartigen Verwendung von *Lactobacillus buchneri* bei der Erhöhung der Fertilität eines weiblichen Säugetiers mittels intrauteriner Applikation des *Lactobacillus buchneri* unabhängig von einer vorherigen zytologischen Untersuchung des Säugetiers gelöst. Dabei weist das Säugetier eine subklinische Endometritis auf, die durch ein Fehlen klinischer Anzeichen einer Endometritis und einen Anteil an polymorphkernigen neutrophilen Granulozyten von mehr als 5 % an der Gesamtzellzahl in einem Ausstrich von Endometriumszellen des Säugetiers gekennzeichnet ist. Die Applikation erfolgt in einem Zeitraum von 20 Tagen bis 100 Tagen postpartum.

Überraschenderweise hat sich gezeigt, dass *Lactobacillus buchneri (L. buchneri)* nicht nur zur Behandlung von klinischer Endometritis geeignet ist, sondern sogar dann, wenn gar keine klinischen Anzeichen einer Endometritis vorliegen, zur Erhöhung der Fertilität des weiblichen Säugetiers verwendet werden kann. Beispielsweise liegen dann, wenn das zu behandelnde weibliche Säugetier eine subklinische Endometritis aufweist oder gänzlich gesund ist, keine klinischen Anzeichen einer Endometritis vor. Demgegenüber sind bei einer klinischen Endometritis definitionsgemäß stets klinische Anzeichen einer Endometritis gegeben. Dabei fokussiert sich die vorliegende Erfindung auf eine Erhöhung der Fertilität bei Tieren, die an einer subklinischen Endometritis erkrankt sind.

Die klinische Endometritis wird nach Sheldon et al. (2006) als das Vorhandensein von purulentem (> 50 % Eiter), uterinem Ausfluss in der Vagina nach 21 oder mehr Tagen postpartum oder mukopurulentem (ungefähr 50 % Eiter, 50 % Schleim) Ausfluss in der Vagina nach 26 Tagen postpartum definiert. Dabei ist das Allgemeinbefinden der betroffenen Kühe ungestört und die Körperinnentemperatur nicht erhöht. Als weiteres Diagnosekriterium für die chronische Endometritis wird von LeBlanc et al. (2002) ein Zervixdurchmesser von mehr als 7,5 cm nach 20 Tagen postpartum aufgeführt.

Bei der subklinischen Endometritis handelt es sich um eine Entzündung des Endometriums, die sich durch das Fehlen klinisch apparenter Symptome auszeichnet. Im Unterschied zur klinischen Endometritis zeigen betroffene Tiere keinen eitrigen vaginalen Ausfluss bei ungestörtem Allgemeinbefinden (Sheldon et al. 2006). Die subklinische Endometritis lässt sich aufgrund der fehlenden klinischen Symptomatik mit Hilfe der klinischen Diagnostik nicht feststellen, dafür bedarf es weitergehender Diagnosemethoden wie der Zytologie. Dabei zeigt ein erhöhter Anteil an polymorphkernigen neutrophilen Granulozyten (PMN) im Ausstrich von Endometriumszellen das Vorhandensein einer Entzündung im Endometrium an. Gängige Methoden für die Entnahme von Endometriumszellen sind der Abstrich mittels Cytobrush (Kasimanickam *et al.* 2004) oder die uterine Lavage (Gilbert et al. 2005). Im Rahmen der vorliegenden Erfindung wird zur Unterscheidung zwischen an subklinischer Endometritis erkrankten Tieren und gesunden Tieren in Übereinstimmung mit der Literatur ein Grenzwert für den PMN-Gehalt in einem zytologischen Präparat (Endometriumsausstrich) von 5 % der Gesamtzellzahl angesetzt (Gilbert et al. 2005; Gabler et al. 2009; Fischer et al. 2010; Lima et al. 2013). Ist der PMN-Gehalt größer als 5 %, liegt eine subklinische Endometritis vor. Ist er kleiner oder gleich 5 %, liegt keine subklinische Endometritis vor.

Schließlich ist zu beachten, dass die Metritis eine von dem Krankheitsbild der subklinischen Endometritis unterschiedliche Erkrankung ist. Das klinische Erscheinungsbild der akuten, puerperalen Metritis zeigt sich in einem abnorm vergrößertem Uterus und einem übelriechenden, wässrigen, rot-braunen uterinen Ausfluss innerhalb 21 Tagen postpartum. Kühe, die an Metritis leiden, zeigen ein gestörtes Allgemeinbefinden und weitere Symptome einer systemischen Erkrankung wie verminderte Milchleistung oder andere Anzeichen einer Toxämie. Die Körperinnentemperatur beträgt mehr als 39,5°C (Sheldon et al. 2006).

Die erfindungsgemäß beanspruchte neuartige Verwendung kann daher auch als die Verwendung von *Lactobacillus buchneri* zur Erhöhung der Fertilität eines weiblichen Säugetiers, das an einer subklinischen Endometritis erkrankt ist, aber keine Anzeichen einer klinischen Endometritis zeigt, formuliert werden.

Die Anwendung von *Lactobacillus buchneri* als probiotische Bakterien stellt eine Alternative zum bisher durchgeführten Antibiotikaeinsatz dar. Der erfindungsgemäß eingesetzte probiotische Stamm verdrängt als nichtpathogener Keim einerseits die pathogenen Keime von der Gebärmutterschleimhaut und unterstützt andererseits die Wiederherstellung einer schützenden kommensalen Mikroflora auf der Gebärmutterschleimhaut. Durch diese neuartige Antibiotika-freie Behandlungsalternative wird also der Aufbau einer schützenden kommensalen Mikroflora im Uterus unterstützt, um so insbesondere nachhaltig leistungsfähige und gesunde Zuchttiere zu erhalten. Die Vermeidung des Einsatzes von Antibiotika trägt zum gesundheitlichen Verbraucherschutz bei, indem die Entstehung von Resistenzen gegenüber Antibiotika minimiert wird.

Ausgehend von dem existierenden Stand der Technik war es vollkommen überraschend, dass eine Erhöhung der Fertilität durch eine intrauterine Behandlung mit *Lactobacillus buchneri* erreicht werden kann, wenn keine klinischen Anzeichen einer Endometritis vorliegen, sondern die behandelten Tiere an einer subklinischen Endometritis erkrankt sind. Denn bislang war man davon ausgegangen, dass die klinisch ausgeprägte Endometritis eine der Hauptursachen für eine verringerte Fertilität bei weiblichen Säugetieren darstellt. Daher wurden bei den aus dem Stand der Technik bekannten Verfahren in der Regel nur solche Tiere behandelt, die klinische Anzeichen einer Endometritis zeigten. Aber auch Tiere, die Anzeichen einer subklinischen Endometritis zeigen, haben verminderte Fruchtbarkeitsraten. Bislang gibt es keine erfolgreiche Therapiestrategie, die neben einer Heilung mit einer erhöhten Fruchtbarkeit einhergeht. Demgegenüber gestattet es die vorliegend beanspruchte neuartige Verwendung von *Lactobacillus buchneri,* nun alle weiblichen Säugetiere ohne Anzeichen einer klinischen Endometritis unabhängig von einer vorherigen zytologischen Untersuchung mit *Lactobacillus buchneri* zu behandeln, um eine Erhöhung der Fertilität zu erreichen. Dabei ist der Effekt einer Fertilitätserhöhung umso ausgeprägter, je schwerwiegender eine subklinische Erkrankung des behandelten Tieres ist, die zu einer vorherigen Fertilitätsverringerung geführt hat.

Aus dem Stand der Technik ist es bekannt, dass eine intravaginale Applikation mit Laktobazillen sowohl einen Erfolg auf die Behandlung von Krankheiten als auch bei der Erhöhung der Fruchtbarkeit haben kann. Aber kürzlich konnte gezeigt werden (Genis et al. 2017), dass vaginal applizierte Laktobazillen nicht das Endometrium erreichen. Somit weisen vaginal applizierte Laktobazillen einen vollkommen anderen Wirkmechanismus als intrauterin applizierte Laktobazillen auf, was sich auch in einer signifikant unterschiedlichen Erhöhung der Fertilität zeigt, wie weiter unten dargelegt werden wird.

Als Erhöhung der Fertilität wird insbesondere eine Verkürzung der Güstzeit, eine Verkürzung der Rastzeit und/oder eine Verringerung der Anzahl der künstlichen Besamungen, die zum Trächtigwerden des weiblichen Säugetiers erforderlich sind, verstanden. Die Güstzeit bezeichnet dabei den Zeitraum zwischen der Geburt eines Nachkommen und dem ersten Trächtigkeitstag einer nachfolgenden Trächtigkeit. Die Rastzeit bezeichnet die Zeit zwischen der Geburt eines Nachkommen und dem Tag der ersten Besamung. Bei Rindern wird die Geburt eines Nachkommen auch als Abkalbung bezeichnet. Bei einer Sau wird die Geburt eines Nachkommen auch als Abferkelung bezeichnet.

In einer Variante wird die Güstzeit durch die erfindungsgemäß beanspruchte Verwendung von *Lactobacillus buchneri* um mindestens 10 Tage, insbesondere um mindestens 15 Tage, insbesondere um mindestens 20 Tage, insbesondere um mindestens 25 Tage, insbesondere um mindestens 30 Tage, insbesondere um mindestens 35 Tage, insbesondere um mindestens 40 Tage, insbesondere um mindestens 45 Tage, insbesondere um mindestens 50 Tage, insbesondere um mindestens 55 Tage, insbesondere um mindestens 60 Tage, insbesondere um mindestens 65 Tage, insbesondere um mindestens 70 Tage, insbesondere um mindestens 75 Tage, insbesondere um mindestens 80 Tage, insbesondere um mindestens 85 Tage, insbesondere um mindestens 90 Tage, insbesondere um mindestens 95 Tage, insbesondere um mindestens 100 Tage verkürzt. Dabei kann die Verkürzung beispielsweise 10 bis 100 Tage, 15 bis 95 Tage, 20 bis 90 Tage, 25 bis 85 Tage, 30 bis 80 Tage, 35 bis 75 Tage, 40 bis 70 Tage, 45 bis 65 Tage oder 50 bis 60 Tage betragen. Die Verkürzung der Güstzeit um die vorgenannten Zeiten bzw. Zeiträume ist insbesondere bei einer Erhöhung der Fertilität einer Kuh als weiblichem Säugetier anzuwenden.

In einer Variante wird die Rastzeit um mindestens 5 Tage, insbesondere um mindestens 10 Tage, insbesondere um mindestens 15 Tage und ganz besonders um mindestens 20 Tage verkürzt. Beispielsweise kann die Rastzeit um 5 bis 20 Tage, insbesondere um 10 bis 15 Tage verkürzt werden. Auch die Verkürzung der Rastzeit um die vorgenannten Zeiten bzw. Zeiträume ist insbesondere bei einer Erhöhung der Fertilität einer Kuh als weiblichem Säugetier anzuwenden.

In einer Variante lässt sich die Anzahl der bis zum Trächtigwerden der Tiere erforderlichen künstlichen Besamungen statistisch um mindestens 0,1, insbesondere um mindestens 0,2, insbesondere um mindestens 0,3, insbesondere um mindestens 0,4, insbesondere um mindestens 0,5, insbesondere um mindestens 0,6, insbesondere um mindestens 0,7, insbesondere um mindestens 0,8, insbesondere um mindestens 0,9, insbesondere um mindestens 1,0, insbesondere um mindestens 1,1, insbesondere um mindestens 1,2, insbesondere um mindestens 1,3, insbesondere um mindestens 1,4, insbesondere um mindestens 1,5, insbesondere um mindestens 1,6, insbesondere um mindestens 1,7, insbesondere um mindestens 1,8, insbesondere um mindestens 1,9, und ganz besonders um mindestens 2,0 reduzieren. Beispielsweise kann eine statistische Reduktion der Anzahl der künstlichen Besamungen um 0,1 bis 2,0, insbesondere um 0,2 bis 1,9, insbesondere um 0,3 bis 1,8, insbesondere um 0,4 bis 1,7, insbesondere um 0,5 bis 1,6, insbesondere um 0,6 bis 1,5, insbesondere um 0,7 bis 1,4, insbesondere um 0,8 bis 1,3, insbesondere um 0,9 bis 1,2, insbesondere um 1,0 bis 1,1 erreicht werden. Auch diese Verringerung der Anzahl der erforderlichen künstlichen Besamungen als Beispiel der Erhöhung der Fertilität ist insbesondere bei einer Erhöhung der Fertilität einer Kuh als weiblichem Säugetier anzuwenden.

In einer Variante handelt es sich bei dem weiblichen Säugetier, das keine klinischen Anzeichen einer Endometritis zeigt und an einer subklinischen Endometritis leidet, um ein weibliches Säugetier, das eine gegenüber einer vergleichbaren Standardpopulation weiblicher Säugetiere verringerte Fertilität aufweist. Diese verringerte Fertilität kann sich beispielsweise in einer gegenüber der Standardpopulation verlängerten Güstzeit, verlängerten Rastzeit oder höheren Anzahl erforderlicher künstlicher Besamungen bis zum erfolgreichen Trächtigwerden widerspiegeln.

Bei dem zu behandelnden weiblichen Säugetier handelt es sich insbesondere um ein nichtmenschliches Säugetier, und zwar insbesondere um ein Säugetier der Ordnung der Paarhufer oder der Unpaarhufer. Zur Behandlung mit *Lactobacillus buchneri* besonders geeignete Familien dieser Ordnungen sind die Familien der Pferde, der Hornträger und der Echten Schweine. In einer Variante handelt es sich bei dem zu behandelnden weiblichen Säugetier um ein Säugetier, das einer künstlichen Besamungen unterzogen werden soll, beispielsweise um ein Zuchtsäugetier.

In einer Variante handelt es sich bei dem weiblichen Säugetier um eine Kuh (also ein weibliches Rind) wie etwa eine Milchkuh, eine Sau (also ein weibliches Schwein) oder eine Stute (also ein weibliches Pferd).

In einer Variante weist der verwendete *Lactobacillus buchneri* eine DNA-Sequenz auf, die zu mindestens 96 %, insbesondere zumindest in 97 %, insbesondere zu mindestens 97,5 %, insbesondere zu mindestens 98 %, insbesondere zu mindestens 98,5 %, insbesondere zu mindestens 99 %, insbesondere zu mindestens 99,5 %, insbesondere zu mindestens 99,9 % identisch zu der DNA-Sequenz der SEQ ID NO: 1 ist. Bei dieser DNA-Sequenz handelt es sich um die DNA des Lactobacillus-buchneri-Stamms NRRL B-30929. Diese DNA-Sequenz weist die Genbank-Zugangsnummer CP002652.1 auf. Details zu dieser DNA-Sequenz sowie die vollständige Sequenz sind beispielsweise über folgende Internetseite abrufbar: ncbi.nlm.nih.gov/nuccore/329127738.

In einer Variante ist die DNA-Sequenz des eingesetzten *Lactobacillus buchneri* zu 95 % bis 100 %, insbesondere 96 % bis 99,9 %, insbesondere 97 % bis 99,5 %, insbesondere 98 % bis 99 % und ganz besonders 98,5 % bis 99,5 % identisch zu der vorgenannten DNA-Sequenz.

In einer Variante weist der verwendete *Lactobacillus buchneri* eine DNA-Sequenz auf, die zu mindestens 96 %, insbesondere zumindest in 97 %, insbesondere zu mindestens 97,5 %, insbesondere zu mindestens 98 %, insbesondere zu mindestens 98,5 %, insbesondere zu mindestens 99 %, insbesondere zu mindestens 99,5 %, insbesondere zu mindestens 99,9 % identisch zu der DNA-Sequenz der SEQ ID NO: 2 ist. Bei dieser DNA-Sequenz handelt es sich um die DNA des Lactobacillus-buchneri-Stamms CD034. Diese DNA-Sequenz weist die Genbank-Zugangsnummer CP003043.1 auf. Details zu dieser DNA-Sequenz sowie die vollständige Sequenz sind beispielsweise über folgende Internetseite abrufbar: ncbi.nlm.nih.gov/nuccore/cp003043.

In einer Variante ist die DNA-Sequenz des eingesetzten *Lactobacillus buchneri* zu 95 % bis 100 %, insbesondere 96 % bis 99,9 %, insbesondere 97 % bis 99,5 %, insbesondere 98 % bis 99 % und ganz besonders 98,5 % bis 99,5 % identisch zu der vorgenannten DNA-Sequenz.

In einer Variante ist die DNA-Sequenz des eingesetzten *Lactobacillus buchneri* zu 95 % bis 100 %, insbesondere 96 % bis 99,9 %, insbesondere 97 % bis 99,5 %, insbesondere 98 % bis 99 % und ganz besonders 98,5 % bis 99,5 % identisch zu der vorgenannten DNA-Sequenz.

In einer Variante weist der verwendete *Lactobacillus buchneri* eine DNA-Sequenz auf, die zu mindestens 96 %, insbesondere zumindest in 97 %, insbesondere zu mindestens 97,5 %, insbesondere zu mindestens 98 %, insbesondere zu mindestens 98,5 %, insbesondere zu mindestens 99 %, insbesondere zu mindestens 99,5 %, insbesondere zu mindestens 99,9 % identisch zu der DNA-Sequenz des Lactobacillus-buchneri-Stamms ist, der bei der Leibniz-Institut DSMZ-Deutsche Sammlung von Mikroorganismen und Zellkulturen GmbH unter der DSMZ-Aufnahmenummer DSM 32407 am 21. Dezember 2016 im Namen der Freien Universität Berlin hinterlegt wurde.

In einer Variante ist die DNA-Sequenz des eingesetzten *Lactobacillus buchneri* zu 95 % bis 100 %, insbesondere 96 % bis 99,9 %, insbesondere 97 % bis 99,5 %, insbesondere 98 % bis 99 % und ganz besonders 98,5 % bis 99,5 % identisch zu der DNA-Sequenz des hinterlegten Stamms.

In einer Variante wird der *Lactobacillus buchneri* insbesondere in einem wässrigen Trägermedium, wie beispielsweise einer geeigneten Pufferlösung, dem zu behandelnden weiblichen Säugetier intrauterin appliziert. Ein geeignetes Applikationsvolumen beträgt dabei 5 ml bis 100 ml, insbesondere 10 ml bis 90 ml, insbesondere 15 ml bis 80 ml, insbesondere 20 ml bis 70 ml, insbesondere 30 ml bis 60 ml und ganz besonders 40 ml bis 50 ml. Ein Applikationsvolumen von 15 bis 25 ml, also von rund 20 ml, ist besonders geeignet. Ein besonders geeignetes wässriges Trägermedium ist eine isotonische Kochsalzlösung. Eine derartige Kochsalzlösung weist einen Kochsalzgehalt in Höhe von 0,9 % (m/v) auf. Geeignete Mengen an zu applizierenden *Lactobacillus buchneri* liegen im Bereich von 1*10⁹ bis 1*10¹¹ koloniebildenden Einheiten (CFU), insbesondere 5*10⁹ bis 5*10¹⁰ CFU, insbesondere 8*10⁹ bis 3*10¹⁰ CFU, insbesondere 1*10¹⁰ bis 2*10¹⁰ CFU.

In einer Variante wird das weibliche Säugetier in einem Zeitraum von 20 bis 90 Tagen postpartum (pp) mit dem *Lactobacillus buchneri* behandelt, insbesondere in einem Zeitraum von 22 bis 90 Tagen pp, insbesondere in einem Zeitraum von 24 bis 90 Tagen pp, insbesondere in einem Zeitraum von 26 bis 90 Tagen pp, insbesondere in einem Zeitraum von 28 bis 90 Tagen pp, insbesondere in einem Zeitraum von 30 bis 90 Tagen pp, insbesondere in einem Zeitraum von 35 bis 90 Tagen pp, insbesondere in einem Zeitraum von 40 bis 80 Tagen pp, insbesondere in einem Zeitraum von 50 bis 70 Tagen pp und ganz besonders in einem Zeitraum von 55 bis 60 Tagen pp. Weitere geeignete Behandlungszeiträume sind 22 bis 38 Tage pp, 24 bis 36 Tage pp, 26 bis 34 Tage pp und 28 bis 30 Tage pp.

Hierdurch offenbart wird ein medizinisches Verfahren zur Behandlung eines weiblichen Säugetiers, das keine klinischen Anzeichen einer Endometritis zeigt, aber an einer subklinischen Endometritis leidet (definiert durch einen PMN-Gehalt > 5 % an der Gesamtzellzahl in einem Ausstrich von Endometriumszellen) mittels intrauteriner Applikation von *Lactobacillus buchneri* zur Erhöhung der Fertilität. Bei dem behandelten weiblichen Säugetier handelt es sich insbesondere um ein solches weibliches Säugetier, das gegenüber einer Kontrollpopulation eine verringerte Fertilität aufweist.

Ein Aspekt der vorstehend beanspruchten Erfindung betrifft die Verwendung von *Lactobacillus buchneri* als Bestandteil eines nicht-menschlichen Spermienpräparats, also beispielsweise einer Spermiensuspension. Durch die vorgenannten Eigenschaften von *Lactobacillus buchneri* auf die Fertilität weiblicher Säugetiere eignen sich Bakterien der Spezies *Lactobacillus buchneri* hervorragend als ein solcher Bestandteil eines Spermienpräparats. Beispielsweise können diese Bakterien als Verdünner für eine Spermiensuspension oder als Konservierungsmittel eines Spermienpräparats eingesetzt werden. Wenn ein entsprechendes Spermienpräparat mit *Lactobacillus buchneri* versetzt ist, kann in einem Besamungsschritt, der ohnehin durchgeführt werden muss, um ein weiteres Trächtigwerden des weiblichen Säugetiers zu erreichen, eine gleichzeitige Applikation von *Lactobacillus buchneri* mit den oben beschriebenen vorteilhaften Auswirkungen auf die Gebärmutterschleimhaut erfolgen. Es ist dann kein gesonderter Schritt einer Applikation von *Lactobacillus buchneri* erforderlich. Folglich erhöht sich bei einer derartigen Verwendung von *Lactobacillus buchneri* als Bestandteil eines Spermienpräparats der zur Behandlung der entsprechenden weiblichen Säugetiere erforderliche Aufwand nicht. Der positive Effekt auf die Fertilität der weiblichen Säugetiere wird indes dennoch erreicht.

Ein weiterer Aspekt der vorliegend beanspruchten Erfindung bezieht sich auf ein Spermienpräparat, das nicht-menschliche Spermien und Bakterien der Spezies *Lactobacillus buchneri* enthält. Darüber hinaus kann das Spermienpräparat weitere Bestandteile enthalten. Die im Zusammenhang mit der vorstehend erläuterten Verwendung von *Lactobacillus buchneri* als Bestandteil eines Spermienpräparats dargestellten positiven Effekte werden dann durch das entsprechende Spermienpräparat erreicht.

Da *Lactobacillus buchneri* gegen einige Antibiotika resistent ist, wäre es grundsätzlich möglich, ein Antibiotikum in einem entsprechenden Spermienpräparat einzusetzen. Wie eingangs erwähnt, sollte ein vorrangiges Ziel der Landwirtschaft jedoch sein, den Einsatz von Antibiotika auf ein Mindestmaß zu reduzieren. Daher ist das Spermienpräparat in einer Variante frei von Antibiotika.

Die oben erläuterten Varianten und Ausgestaltungen der neuartigen Verwendung von *Lactobacillus buchneri* zur Verwendung bei der Erhöhung der Fertilität eines weiblichen Säugetiers sind in analoger Weise auf die neuartige Verwendung von *Lactobacillus buchneri* als Bestandteil eines Spermienpräparats und auf das vorstehend beschriebene Spermienpräparat übertragbar. Dabei können die einzelnen Varianten und Ausgestaltungen in beliebiger Weise miteinander kombiniert werden.

Weitere Einzelheiten der vorliegenden Erfindung werden nachstehend anhand eines Ausführungsbeispiels und anhand von Figuren näher erläutert. Es zeigen:
- Figur 1A: eine grafische Darstellung des Anteils polymorphkerniger neutrophiler Granulozyten (PMN) eine Woche nach Applikation von *Lactobacillus buchneri* bzw. von steriler Kochsalzlösung als Placebo bei Rindern;
- Figur 1B: eine grafische Darstellung des Anteils polymorphkerniger neutrophiler Granulozyten (PMN) drei Wochen nach Applikation von *Lactobacillus buchneri* bzw. von steriler Kochsalzlösung als Placebo bei Rindern;
- Figur 2A: die normalisierte mRNA-Expression des Gens CXCL1/2 drei Wochen nach Applikation von *Lactobacillus buchneri* bzw. physiologischer Kochsalzlösung als Placebo bei Rindern;
- Figur 2B: die normalisierte mRNA-Expression des Gens CXCR2 drei Wochen nach Applikation von *Lactobacillus buchneri* bzw. physiologischer Kochsalzlösung als Placebo bei Rindern;
- Figur 2C: die normalisierte mRNA-Expression des Gens IL1B drei Wochen nach Applikation von *Lactobacillus buchneri* bzw. physiologischer Kochsalzlösung als Placebo bei Rindern;
- Figur 2D: die normalisierte mRNA-Expression des Gens IL8 drei Wochen nach Applikation von *Lactobacillus buchneri* bzw. physiologischer Kochsalzlösung als Placebo bei Rindern und
- Figur 3: die Motilität von Spermien in dem Antibiotika-freien Verdünner Triladyl^{®} ohne Bakterien (nativ) oder in Anwesenheit von *L. mucosae* oder von *L. buchneri.*

### Ausführungsbeispiele

### 1) Isolierung und Charakterisierung probiotischer Milchsäurebakterien-Stämme

Zur Isolierung uteriner Milchsäurebakterien wurden Cytobrush-Proben von 39 gesunden laktierenden Kühen genommen. Diese Proben wurden in MRS-Agar und SL-Agar sowohl anaerob als auch aerob kultiviert. Die meisten Isolate konnten mittels SL-Agar gewonnen werden, wobei alle Keime sowohl anaerob als auch aerob wuchsen. Gram-positive, Katalasenegative, Indol-negative und Nitrate-reduzierend-negative Bakterien wurden als Lactobacilli identifiziert.

Durch eine *Lactobacillus-spezifische* PCR, eine nachfolgende phylogenetische PCR mit 16S-rDNA-Primern und eine anschließende Sequenzierung konnten folgende Spezies näher charakterisiert werden: *L. buchneri, L. ruminis, L. amylovorus, L. plantarum, Weissella (W.) paramesenteroides, Aerococcus* spp. und *Pediococcus* sp.

Nach den Zellkulturexperimenten wurden drei der isolierten Lactobacillus-Stämme, nämlich *L*. *buchneri, L. ruminis* und *L. amylovorus,* auf ihr biochemisches Profil mittels des API-50-CH-System getestet. Dabei traten deutliche Unterschiede im Metabolismus von Kohlenhydraten auf.

Die Sequenzierung eines Teils des Genoms des isolierten und unter der Zugangsnummer DSM 32407 hinterlegten *L.-buchneri*-Stamms ergab eine 732 kB große Sequenz, die eine rund 98%ige Homologie mit dem in der Genbank abgelegten Stamm *Lactobacillus buchneri* CD034 (Genbank-Zugangsnummer CP003043, SEQ ID NO: 2) aufwies. Sie ist im beigefügten Sequenzprotokoll als SEQ ID NO: 3 aufgelistet.

Die In-vitro-Wirkung der verschiedenen Milchsäurebakterien-Stämme wurde näher charakterisiert. Die diesbezüglichen Ergebnisse wurden bereits veröffentlicht (Gärtner et al. 2015).

### 2) Tiermodell: Behandlung von Milchkühen mit subklinischer Endometritis

### a) Grundlegende Versuchsanordnung

Nach den bereits veröffentlichten In-vitro-Experimenten (Einfluss verschiedener Milchsäurebakterien-Stämme auf die Vitalität und das mRNA-Expressionsprofil endometrialer Epithelzellen) wurde der zuvor isolierte bakterielle Stamm *L. buchneri* für weitergehende Invivo-Untersuchungen ausgewählt.

Zur Ermittlung eines positiven Effekts einer *L.*-*buchneri*-Applikation auf die Fertilität von Milchkühen wurde ein genehmigter Tierversuch (Landesamt für Umwelt, Gesundheit und Verbraucherschutz Brandenburg V3-2347-2-2012 und V3-2347-19-2013) durchgeführt.

Nach einer eingehenden Untersuchung (Vaginoskopie, Ultraschall) wurden die Milchkühe mit subklinischer Endometritis (Lutealphase oder azyklisch) im Zeitraum Tag 24-30 pp zufällig in zwei Gruppen (n=16 Tiere pro Gruppe) unterteilt. Die Tiere der ersten Gruppe wurden mit mit ca. 1-2 × 10¹⁰ koloniebildenden Einheiten des *L.-buchneri*-Stammes in 20 ml einer physiologischen Kochsalzlösung intrauterin behandelt. Die Tiere der Kontrollgruppe bekamen 20 ml der physiologischen Kochsalzlösung als Placebo-Behandlung.

Der isolierte und näher charakterisierte Stamm *L. buchneri* wurde in 15% Glycerin in MRS-Medium (Sigma-Aldrich, Steinheim) für die Langzeit-Lagerung bei -80°C eingefroren (Gärtner et al. 2015). Aus dieser Stammlösung wurde *L. buchneri* aerob in MRS-Medium (Sigma-Aldrich, Steinheim) bei 37°C für 48 Stunden kultiviert, bis er eine optische Dichte von nahezu eins bei einer Wellenlänge von 600 nm erreichte. Nach Zentrifugation für 10 min bei 15000*g und Resuspendierung in MRS-Medium mit 50 % Glycerin wurden Aliquots von je 200 µl bei -80°C bis zur weiteren Verwendung gelagert.

36-48 Stunden vor Herstellung einer zur intrauterinen Applikation vorgesehenen Lactobacillus-Suspension in isotonischer steriler Kochsalzlösung wurden die Lactobacillus-Aliquots aus dem -80°C-Gefrierschrank entnommen und bei Raumtemperatur aufgetaut, um die koloniebildenden Einheiten pro Milliliter (CFU/ml) zu bestimmen. Die dafür hergestellten Verdünnungsreihen wurden mikroaerophil (Anaerocult C, Merck, Darmstadt) auf Rogosa-SL-Agar (Sigma-Aldrich) bei 37°C kultiviert. Aufgetaute Aliquots wurden bis zur Bestimmung der CFU/ml bzw. bis zur Verdünnung mit isotonischer steriler Kochsalzlösung bei 4 °C gelagert.

Durch ein entsprechendes Experiment wurde festgestellt, dass die Anzahl der CFU/ml in den aufgetauten Aliquots (gelagert bei 4 °C) bis zu 48 Stunden und in den verdünnten Suspensionen (gelagert bei Raumtemperatur) für 8 Stunden stabil waren.

*L*.*-buchneri*-Suspensionen für die intrauterine Applikation enthielten ca. 1,5-2*10¹⁰ koloniebildende Einheiten (CFU) und wurden dann in 20 ml isotonischer steriler Kochsalzlösung (0.9 % (w/v); B. Braun, Melsungen) aufgenommen. Diese *Lactobacillus*-Suspension wurde in eine 20-ml-Plastikspritze (Injekt, B. Braun) aufgezogen. Die zur Applikation vorgesehene verdünnte Suspension wurde immer am Tag der Applikation morgens um 8 Uhr hergestellt, anschließend bei Raumtemperatur zum landwirtschaftlichen Betrieb transportiert und spätestens 8 Stunden nach Herstellung appliziert.

Die als Versuchstiere dienenden Milchkühe wurden am Tag 24-30 pp untersucht und behandelt. Dieser Zeitpunkt wurde ausgewählt, weil hier eine subklinische Endometritis am wahrscheinlichsten zu detektieren ist, die nicht wieder spontan verschwindet. Da über einen Zeitraum von drei Wochen Proben genommen wurden, fand bei dieser Versuchsplanung die letzte Probennahme kurz vor der geplanten Insemination statt. Dies hatte den zusätzlichen Vorteil, dass von den Versuchstieren eine genaue Analyse des Endometriums vor der Insemination erhalten wurde, was besonders in Bezug auf die Fruchtbarkeit als günstig zu bewerten ist.

Zuerst wurden die Tiere durch transrektale Palpation des Uterus beurteilt und mit Ultraschall weiter eingehend untersucht. Daran anschließend wurde mit einem Spekulum eine vaginale Untersuchung durchgeführt, um Tiere mit Anzeichen einer klinisch apparenten Endometritis, d.h. mit mucopurulenten, vaginalem Ausfluss, von der weiteren Untersuchung auszuschließen.

### b) Durchführung der Behandlung

Von klinisch unauffälligen Tieren wurden mit der Cytobrush-Technik (Gabler et al. 2009; Kasimanickam et al. 2005) zytologische Proben genommen. Zunächst wurde ein 60 cm langer Metallkatheter mit einem Durchmesser von 5 mm, der von einer Plastikhülle umgeben war, vaginal eingeführt und unter rektaler Kontrolle durch die Zervix bis in den Uterus vorgeschoben. Ein Cytobrush wurde durch den im Uterus befindlichen Metallkatheter eingeführt und eine Endometriumsprobe entnommen. Das Cytobrush wurde durch den Katheter zurückgezogen und aus dem Uterus entfernt. Auf diese Weise wurden von jedem Tier drei Proben aus der Uterusschleimhaut entnommen. Der Metallkatheter verblieb während der Probenentnahme im Uterus und diente für das Cytobrush als Führungsschiene.

Durch diesen Metallkatheter wurde nun ein steriler 62 cm langer Polytetrafluorethylen-Schlauch mit einem inneren Durchmesser von 2 mm für die aseptische Applikation von *L*. *buchneri* oder von Placebo eingeführt. Die Tiere der ersten Gruppe wurden mit ca. 1,5-2,0*10¹⁰ CFU des *L.-buchneri*-*Stamms* in 20 ml einer physiologischen Kochsalzlösung intrauterin behandelt. Die Tiere der Kontrollgruppe bekamen 20 ml der physiologischen Kochsalzlösung als Placebo-Behandlung.

Anhand des Anteils an polymorphkernigen neutrophilen Granulozyten (PMN) im anschließenden Ausstrich wurden die Kühe als subklinisch erkrankt (PMN > 5 % an der Gesamtzellzahl) oder gesund (PMN ≤ 5 % an der Gesamtzellzahl) eingeteilt.

### c) Überprüfung des Gesundheitszustandes und der Fertilität der Versuchstiere

Zur Beurteilung der Fertilität wurden *L. buchneri*- (n=42) und Placebo-Behandlungen (n=45) von Tieren verglichen. Diese erhöhte Tierzahl ist der Tatsache geschuldet, dass initial mehr Tiere behandelt wurden, da erst nach Applikation festgestellt werden konnte, ob die Tiere an subklinischer Endometritis erkrankt oder gesund waren. Beide Gruppen enthielten somit gesunde und kranke Tiere.

Die Güstzeit war bei den mit *L. buchneri* behandelten Tieren um durchschnittlich 30 Tage signifikant (P = 0,038) kürzer als bei den mit Placebo behandelten Kühen (103 versus 133 Tagen).

Die Rastzeit war bei den mit *L. buchneri* behandelten Tieren ebenfalls kürzer (67 versus 81 Tage).

Es mussten bei den mit *L. buchneri* behandelten Tieren zudem insgesamt weniger künstliche Besamungen vorgenommen werden (1,9 versus 2,4), bis die Tiere trächtig wurden (P = 0,087).

Wenn man bis Tag 200 nach der Abkalbung eine statistische Analyse der Kaplan-Meier-Überlebensrate anwendet, wurden die Kühe mit der *L.*-*buchneri*-Applikation früher tragend als die Tiere der Kontrollgruppe (P = 0,035).

Die bakteriologischen Befunde zeigten keine Auffälligkeiten zwischen Behandlungen mittels *L. buchneri* und Placebo. Weiterhin zeigte sich, dass fast alle Kühe sowohl aus der Placebo-Gruppe als auch aus der *L.-buchneri*-*Gruppe* innerhalb von drei Wochen einen PMN-Anteil von unter 5 % aufwiesen und es im klinischen Erscheinungsbild keine offensichtlichen Unterschiede zwischen den Gruppen gab.

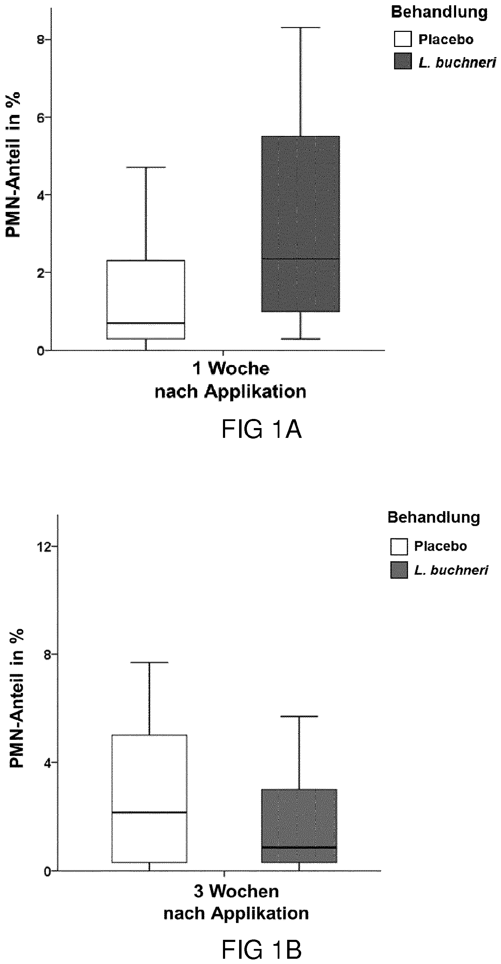

In den Figuren 1A und 1B ist dargestellt, dass sowohl die mit Placebo als auch die mit *L*. *buchneri* behandelte Gruppe der Versuchstiere innerhalb einer Woche nach Applikation einen PMN-Anteil in den untersuchten Zytologieproben des Endometriums von unter 5% aufwiesen. Im klinischen Erscheinungsbild gab es keine offensichtlichen Unterschiede zwischen den beiden Gruppen.

Trotzdem wurde festgestellt, dass die Applikation von *L. buchneri* nach einer Woche einen leicht erhöhten PMN-Anteil im Vergleich mit der Placebo-Behandlung (P=0.071) zur Folge hatte. Ebenso zeigte die Auswertung der Biopsie-Proben eine Woche nach Behandlung, dass neben mononukleären Zellen auch PMNs im Gegensatz zu den Placebo-Tieren in geringer Zahl einwanderten.

Von allen Tieren wurden parallel RNA-Proben für molekularbiologische Analysen gewonnen und aufgearbeitet. Dabei zeigte sich, dass nach einer Woche in den Proben der mit *L. buchneri* behandelten Tiere der Tumornekrosefaktor und in Tendenzen die Gene *PTGS2* und *CXCL5* gegenüber den Proben der mit Placebo behandelten Tiere erhöht exprimiert waren. Diese Daten sind hervorragend in Einklang zu bringen mit den erhaltenen Daten des PMN-Anteils, welcher auch in den mit *L. buchneri* behandelten Tieren am Tag 7 leicht erhöht war.

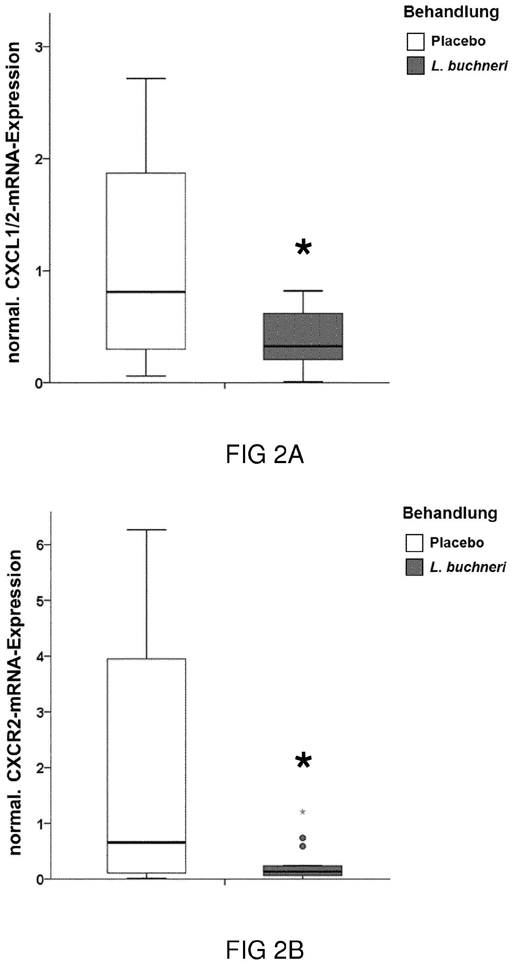

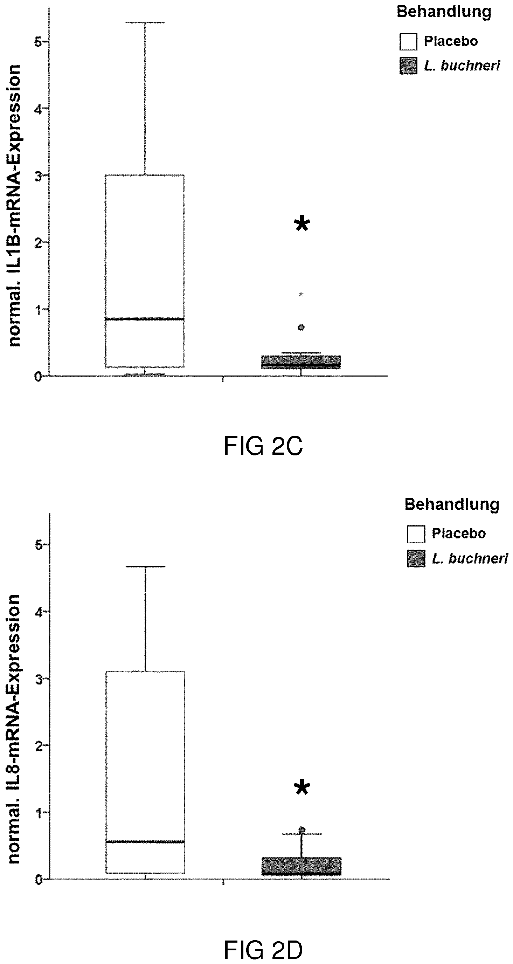

Nach 3 Wochen waren in den mit *L. buchneri* behandelten Kühen die folgenden proinflammatorischen Gene signifikant niedriger exprimiert als in den Placebo-Tieren: *CXCL1*/*2*, *CXCR2, IL1B* und *IL8.* Dies ist in den Figuren 2A bis 2D im Detail dargestellt, die eine normalisierte mRNA-Expression in Proben von mit Placebo und mit *L. buchneri* behandelten Tieren drei Wochen nach Applikation zeigen. Die verbesserten Reproduktionsparameter der mit *L. buchneri* behandelten Tiere werden durch diese molekularbiologischen Ergebnisse unterstützt; *L. buchneri* beugt Entzündungsreaktionen effektiv vor und ist in der Lage, bestehende Entzündungen zu heilen.

Zusammenfassend kann festgehalten werden, dass der eingesetzte Stamm des *L. buchneri* zunächst eine schwache Immunantwort auslöst, dann aber sehr bald zu einer besseren Uterusgesundheit beiträgt, die sich in einer signifikant erhöhten Fertilität manifestiert.

Wie die vorstehend erläuterten Daten zeigen, konnte eine Erhöhung der Fertilität bei allen Tieren beobachtet werden, die keine klinischen Anzeichen einer Endometritis aufweisen. Betrachtet man aus der Gruppe dieser Tiere nun einzig die Tiere, die an einer subklinischen Endometritis litten (PMN-Gehalt > 5 %), tritt der positive Effekt der Behandlung mit *Lactobacillus buchneri* noch weitaus stärker zu Tage.

Zur Beurteilung der Fertilität in der Gruppe der an subklinischer Endometritis erkrankten Tiere wurden *L.-buchneri-*Behandlungen (n = 16) und Placebo-Behandlungen (n = 17) miteinander verglichen.

Die Güstzeit war bei den mit *L. buchneri* behandelten Tieren um durchschnittlich 90 Tage signifikant (P = 0,001) kürzer als bei den mit Placebo behandelten Kühen (74 versus 164 Tagen). Diese Verkürzung um 90 Tage ist dabei dreimal so groß wie die bei allen behandelten Tieren beobachtete durchschnittliche Verkürzung der Güstzeit um 30 Tage. Es war überraschend, dass eine intrauterine Applikation von *Lactobacillus buchneri* einen derart ausgeprägten Effekt auf die Güstzeit von Tieren, die an subklinischer Endometritis erkrankt sind, hat.

Die Rastzeit war bei den mit *L. buchneri* behandelten Tieren vergleichbar mit der der mit Placebo behandelten Kühe (63 versus 65 Tage).

Es mussten bei den mit *L. buchneri* behandelten Tieren zudem insgesamt weniger künstliche Besamungen vorgenommen werden (1,7 versus 3,3), bis die Tiere trächtig wurden (P = 0,012).

Wenn man bis Tag 200 nach der Abkalbung eine statistische Analyse der Kaplan-Meier-Überlebensrate anwendet, wurden die Kühe mit der *L.*-*buchneri*-Applikation früher tragend als die Tiere der Kontrollgruppe (P = 0,001).

Diese Daten sind überraschend besser als die aus dem Stand der Technik bekannten Daten zu vaginal behandelten Kühen, bei denen je nach Behandlungsregime keine Verkürzung der Güstzeit oder eine Verkürzung der Güstzeit um maximal 40 Tage sowie eine Erhöhung der erforderlichen künstlichen Besamungen beobachtet werden konnte (Deng et al. 2015).

Im Rahmen des durchgeführten Versuchs musste eine Festlegung der Wartezeit auf Fleisch und Milch beim Bundesamt für Verbraucherschutz und Lebensmittelsicherheit (BVL) beantragt werden. Das BVL legte eine Wartezeit für Milch und Fleisch auf NULL Tage für den applizierten *L.-buchneri-*Stamm fest. Das heißt, der Einsatz von *L. buchneri* zur Erhöhung der Fertilität hat - im Gegensatz zur bislang üblichen Antibiotika-Behandlung, die eine Wartezeit nach sich zieht - keine finanziellen Einbußen für die Landwirte zur Folge. Damit resultieren aus dem Einsatz von *L. buchneri* zur Erhöhung der Fertilität weiblicher Säugetiere eine Effizienzsteigerung und somit eine spürbare Verbesserung der Wettbewerbsfähigkeit der Milch- und Fleischproduktion.

### 3) Gemeinsame Inkubation von Laktobazillen mit Spermien

Um eine weitere neuartige Verwendungsmöglichkeit von Lactobacilli zu testen, wurde eine Antibiotika-freie Lagerung von Spermienportionen unter Zugabe von Lactobacilli untersucht.

Zur Isolierung von Milchsäurebakterien im nativen Sperma wurden Proben von 25 gesunden Jungbullen verwendet. Diese Proben wurden auf für Milchsäurebakterien selektivem Agar sowohl anaerob als auch aerob kultiviert. Die so isolierten Keime wurden aufgrund der 16S-rRNA-PCR mit phylogenetischen Primern näher charakterisiert. Alle Keime, die sich als Stämme der *Lactobacillaceae-Familie* erwiesen, wurden weiter getestet. Weiterhin wurde die Wirkung dieser isolierten kommensalen Keime auf die Vitalität und somit Befruchtungsfähigkeit der Spermien getestet. Ebenso wurde zu diesem Zweck *L. buchneri* DSM 32407 für diese Untersuchungen herangezogen. Dazu wurden die Spermien verschiedener Bullen mit den Laktobazillen-Keimen in verschiedenen Konzentrationen (1×10⁶ und 1×10⁷ Keime) pro 50 µl Frisch-Ejakulat im Antibiotika-freien Verdünner Triladyl^{®} (1000 µl; nach Herstellerangaben enthaltend TRIS, Zitronensäure, Zucker, Puffer, Glycerin und Reinstwasser) zusammen bei 38°C inkubiert. Bis zu 4h nach Zugabe wurden stündlich die unterschiedlichen Motilitätsparameter (Spermvision) aufgezeichnet. Als Negativkontrolle wurde dasselbe Frisch-Ejakulat, das ohne Laktobazillen-Zugabe in der gleichen Weise im Antibiotika-freien Verdünner Triladyl^{®} (1000 µl) bei 38 °C inkubiert wurde, verwendet. Statt Laktobazillen wurde der Negativkontrolle lediglich der für die Laktobazillen verwendete Puffer zu begeben.

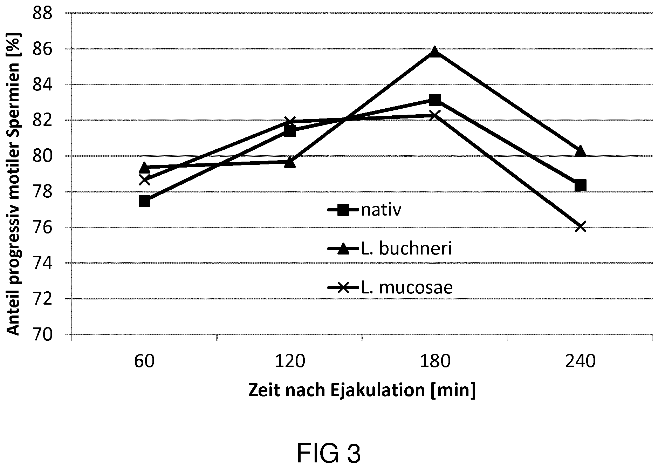

In drei Proben konnten *Lactobacillus-Stämme* isoliert werden, darunter der Stamm *L*. *mucosae. L. mucosae* beeinträchtigte die Motilität der Spermien im Vergleich zu einer bakterienfreien (nativen) Spermiensupension. Demgegenüber konnte durch Zugabe von *Lactobacillus buchneri* eine Erhöhung des Anteils motiler Spermien, insbesondere ab rund 150 Minuten nach Ejakulation, beobachtet werden. Diese Ergebnisse sind in der Figur 3 dargestellt.

Das zeigt, dass *Lactobacillus buchneri* für die Antibiotika-freie Lagerung von Spermien geeignet ist und sowohl gegenüber einer bakterienfreien Lagerung als auch gegenüber einer Ko-Kultur von Spermien mit *L. mucosae* einen deutlich positiven Einfluss auf die Motilität der Spermien hat. Damit eignet sich *Lactobacillus buchneri* sehr gut als Bestandteil eines Spermienpräparats und kann insbesondere zur Erhöhung der Motilität von Spermien in einem In-vitro-Spermienpräparat verwendet werden.

### Liste der in den vorherigen Abschnitten zitierten Literatur

1) Gabler C, Drillich M, Fischer C, Holder C, Heuwieser W & Einspanier R: Endometrial expression of selected transcripts involved in prostaglandin synthesis in cows with endometritis. Theriogenology 71 (2009) 993-1004.
2) Gärtner MA, Bondzio A, Braun N, Jung M, Einspanier R & Gabler C: Detection and characterisation of Lactobacillus spp. in the bovine uterus and their influence on bovine endometrial epithelial cells in vitro. PLoS One 10 (2015) e0119793.
3) Kasimanickam R, Duffield TF, Foster RA, Gartley CJ, Leslie KE, Walton JS & Johnson WH:A comparison of the cytobrush and uterine lavage techniques to evaluate endometrial cytology in clinically normal postparturn dairy cows. Can Vet J 46 (2005) 255-259.
4) LeBlanc SJ, Osawa T & Dubuc J: Reproductive tract defense and disease in postpartum dairy cows. Theriogenology 76 (2011) 1610-1618.
5) Sheldon IM, Cronin JG, Healey GD, Gabler C, Heuwieser W, Streyl D, Bromfield JJ, Miyamoto A, Fergani C & Dobson H: Innate immunity and inflammation of the bovine female reproductive tract in health and disease. Reproduction 148 (2014) R41-51.
6) Sheldon, I. M., Lewis, G. S., LeBlanc, S., Gilbert, R. O.: Defining postpartum uterine disease in cattle. Theriogenology 65 (2006) 1516-1530.
7) Genis S, Bach À, Aris A: Effects of intravaginal lactic acid bacteria on bovine endometrium: Implications in uterine health. Vet Microbiol. 204 (2017) 174-179.
8) Gilbert, R. O., Shin, S. T., Guard, C. L., Erb, H. N., Frajblat, M.: Prevalence of endometritis and its effects on reproductive performance of dairy cows. Theriogenology 64 (2005) 1879-1888.
9) Kasimanickam, R., Duffield, T. F., Foster, R. A., Gartley, C. J., Leslie, K. E., Walton, J. S., Johnson, W. H.: Endometrial cytology and ultrasonography for the detection of subclinical endometritis in postpartum dairy cows. Theriogenology 62 (2004) 9-23.
10) Lima, F. S., Bisinotto, R. S., Ribeiro, E. S., Greco, L. F., Ayres, H., Favoreto, M. G., Carvalho, M. R., Galvao, K. N., Santos, J E P.: Effects of 1 or 2 treatments with prostaglandin F(2)alpha on subclinical endometritis and fertility in lactating dairy cows inseminated by timed artificial insemination. Journal of Dairy Science 96 (2013) 6480-6488.
11) Bhandari, P.; Prabha, V.: Evaluation of profertility effect of probiotic Lactobacillus plantarum 2621 in a murine model. The Indian Journal of Medical Research 142 (2015) 79-84.
12) Deng, Q. et al.: Intravaginally administered lactic acid bacteria expedited uterine involution and modulated hormonal profiles of transition dairy cows. Journal of Dairy Science 98 (2015) 6018-6028.
13) Otero, M. C., Morelli, L., Nader-Macías, M. E.: Probiotic properties of vaginal lactic acid bacteria to prevent metritis in cattle. Letters in Applied Microbiology 43 (2006) 91-97.
14) Barbonetti, A. et al.: Effect of vaginal probiotic lactobacilli on in vitro-induced sperm lipid peroxidation and its impact on sperm motility and viability. Fertility and Sterility 95 (2011) 2485-2488.
15) LeBlanc, S. J., Duffield, T. F., Leslie, K. E., Bateman, K. G., Keefe, G. P., Walton, J. S., Johnson, W. H.: Defining and diagnosing postpartum clinical endometritis and its impact on reproductive performance in dairy cows. Journal of Dairy Science 85 (2002) 2223-2236.
16) S. LIU ET AL.: Complete Genome Sequence of Lactobacillus buchneri NRRL B-30929, a Novel Strain from a Commercial Ethanol Plant. JOURNAL OF BACTERIOLOGY 193 (2011), Nr. 15, Seiten 4019-4020.

## Patentansprüche

1. *Lactobacillus buchneri* zur Verwendung bei der Erhöhung der Fertilität eines weiblichen Säugetiers mittels intrauteriner Applikation des *Lactobacillus buchneri* unabhängig von einer vorherigen zytologischen Untersuchung des Säugetiers, wobei das Säugetier eine subklinische Endometritis aufweist, die durch ein Fehlen klinischer Anzeichen einer Endometritis und einen Anteil an polymorphkernigen neutrophilen Granulozyten von mehr als 5 % an der Gesamtzellzahl in einem Ausstrich von Endometriumszellen des Säugetiers gekennzeichnet ist, wobei die Applikation in einem Zeitraum von 20 Tagen bis 100 Tagen postpartum erfolgt.

2. *Lactobacillus buchneri* zur Verwendung nach Anspruch 1, **dadurch gekennzeichnet, dass** das Säugetier der Ordnung der Paarhufer oder der Unpaarhufer angehört.

3. *Lactobacillus buchneri* zur Verwendung nach Anspruch 2, **dadurch gekennzeichnet, dass** es sich bei dem Säugetier um ein Rind, ein Schwein oder ein Pferd handelt.

4. *Lactobacillus buchneri* zur Verwendung nach einem der vorherigen Ansprüche, **dadurch gekennzeichnet, dass** der *Lactobacillus buchneri* eine DNA-Sequenz aufweist, die zu mindestens 95 % identisch zu der DNA-Sequenz der SEQ ID NO: 1 ist.

5. *Lactobacillus buchneri* zur Verwendung nach einem der vorherigen Ansprüche, **dadurch gekennzeichnet, dass** der *Lactobacillus buchneri* eine DNA-Sequenz aufweist, die zu mindestens 95 % identisch zu der DNA-Sequenz der SEQ ID NO: 2 ist.

6. *Lactobacillus buchneri* zur Verwendung nach einem der vorherigen Ansprüche, **dadurch gekennzeichnet, dass** der *Lactobacillus buchneri* eine DNA-Sequenz aufweist, die zu mindestens 95 % identisch zu der DNA-Sequenz der SEQ ID NO: 3 ist.

7. *Lactobacillus buchneri* zur Verwendung nach einem der vorherigen Ansprüche, **dadurch gekennzeichnet, dass** der *Lactobacillus buchneri* eine DNA-Sequenz aufweist, die zu mindestens 95 % identisch zu der DNA-Sequenz des Lactobacillus-buchneri-Stamms ist, der bei der Leibniz-Institut DSMZ-Deutsche Sammlung von Mikroorganismen und Zellkulturen GmbH unter der DSMZ-Aufnahmenummer DSM 32407 hinterlegt wurde.

8. Verwendung von *Lactobacillus buchneri* als Bestandteil eines nicht-menschlichen Spermienpräparats.

9. Spermienpräparat, umfassend nicht-menschliche Spermien und *Lactobacillus buchneri.*

10. Spermienpräparat nach Anspruch 9, **dadurch gekennzeichnet, dass** es antibiotikafrei ist.

## Claims

1. *Lactobacillus buchneri* for use in increasing the fertility of a female mammal by means of intrauterine application of *Lactobacillus buchneri* independently of a previous cytological examination of the mammal, wherein the mammal has a subclinical endometritis which is **characterized by** the missing of clinical signs of an endometritis and a fraction of polymorphonuclear neutrophil granulocytes of more than 5% in the total cell count in a smear of endometrium cells of the mammal, wherein the application is effected in a period of 20 days to 100 days postpartum.

2. *Lactobacillus buchneri* for use according to claim 1, **characterized in that** the mammal belongs to the order of even-toed ungulates or odd-toed ungulates.

3. *Lactobacillus buchneri* for use according to claim 2, **characterized in that** the mammal is a bovine, a pig or a horse.

4. *Lactobacillus buchneri* for use according to any one of the preceding claims, **characterized in that** the *Lactobacillus buchneri* has a DNA sequence which is identical to the DNA sequence of SEQ ID NO: 1 for at least 95%.

5. *Lactobacillus buchneri* for use according to any one of the preceding claims, **characterized in that** the *Lactobacillus buchneri* has a DNA sequence which is identical to the DNA sequence of SEQ ID NO: 2 for at least 95%.

6. *Lactobacillus buchneri* for use according to any one of the preceding claims, **characterized in that** the *Lactobacillus buchneri* has a DNA sequence which is identical to the DNA sequence of SEQ ID NO: 3 for at least 95%.

7. *Lactobacillus buchneri* for use according to any of the preceding claims, **characterized in that** the *Lactobacillus buchneri* has a DNA sequence which is identical to the DNA sequence of the *Lactobacillus buchneri* strain which has been deposited with the Leibniz Institute DSMZ-Deutsche Sammlung von Mikroorganismen und Zellkulturen GmbH under the DSMZ accession number DSM 32407.

8. Use of *Lactobacillus buchneri* as a component of a non-human sperm preparation.

9. Sperm preparation, comprising non-human sperm and *Lactobacillus buchneri.*

10. Sperm preparation according to claim 9, **characterized in that** it is free of antibiotics.

## Revendications

1. *Lactobacillus buchneri* à utiliser dans l'augmentation de la fertilité d'un mammifère femelle par application intra-utérine de *Lactobacillus buchneri* indépendamment d'un examen cytologique préalable du mammifère, le mammifère présentant une endométrite subclinique, qui est **caractérisée par** l'absence de signes cliniques d'une endométrite et par une proportion de granulocytes neutrophiles polymorphonucléaires supérieure à 5 % du nombre total de cellules dans un frottis de cellules endométriales du mammifère, l'application ayant lieu dans une période de 20 jours à 100 jours post-partum.

2. *Lactobacillus buchneri* à utiliser selon la revendication 1, **caractérisé en ce que** le mammifère appartient à l'ordre des artiodactyles ou des non artiodactyles.

3. *Lactobacillus buchneri* à utiliser selon la revendication 2, **caractérisé en ce que** le mammifère est un boeuf, un porc ou un cheval.

4. *Lactobacillus buchneri* à utiliser selon l'une quelconque des revendications précédentes, **caractérisé en ce que** ledit *Lactobacillus buchneri* a une séquence d'ADN qui est identique à la séquence d'ADN de la SEQ ID NO : 1 pour au moins 95 %.

5. *Lactobacillus buchneri* à utiliser selon l'une quelconque des revendications précédentes, **caractérisé en ce que** ledit *Lactobacillus buchneri* a une séquence d'ADN qui est identique à la séquence d'ADN de la SEQ ID NO : 2 pour au moins 95 %.

6. *Lactobacillus buchneri* à utiliser selon l'une quelconque des revendications précédentes, **caractérisé en ce que** ledit *Lactobacillus buchneri* a une séquence d'ADN qui est identique à la séquence d'ADN de la SEQ ID NO : 3 pour au moins 95 %.

7. *Lactobacillus buchneri* à utiliser selon l'une quelconque des revendications précédentes, **caractérisé en ce que** ledit *Lactobacillus buchneri* a une séquence d'ADN qui est identique à la séquence d'ADN de la souche de *Lactobacillus buchneri* déposée auprès de l'Institut Leibniz DSMZ-Deutsche Sammlung von Mikroorganismen und Zellkulturen GmbH sous le numéro d'enregistrement DSMZ DSM 32407.

8. Utilisation de *Lactobacillus buchneri*comme composant d'une préparation de sperme non humain.

9. Préparation de sperme, comprenant du sperme non humain et *du Lactobacillus buchneri.*

10. Préparation de sperme selon la revendication 9, **caractérisée en ce qu'**elle est exempte d'antibiotiques.
